Europäisches Patentamt

European Patent Office

Office européen des brevets

(11) Publication number: **0 544 212 A1**

# EUROPEAN PATENT APPLICATION

(21) Application number: **92119952.7**

(22) Date of filing: **24.11.92**

(51) Int. Cl.⁵: **C12Q 1/68**, G01N 33/58

(30) Priority: **26.11.91 JP 335580/91**

(43) Date of publication of application:
**02.06.93 Bulletin 93/22**

(84) Designated Contracting States:
**CH DE FR GB LI SE**

(71) Applicant: **NISSHIN FLOUR MILLING CO., LTD.**
**19-12, Nihonbashi-koamicho**
**Chuo-ku, Tokyo(JP)**

(72) Inventor: **Sasaki, Kazuyuki**
**7-3-7-404, Hikarigaoka**
**Nerima-ku, Tokyo(JP)**
Inventor: **Sato, Takeya**
**3-4-8, Suehiro-cho, Kawagoe-shi**
**Saitama-ken(JP)**

(74) Representative: **Türk, Gille, Hrabal, Leifert**
**Brucknerstrasse 20**
**W-4000 Düsseldorf 13 (DE)**

(54) **Method for the detection or quantitation of trace substances.**

(57) A highly sensitive method for the detection or quantitation of a trace substance in a sample is disclosed. The method includes reacting a substance to be detected with a specifically binding substance which has a specific affinity thereto and is labeled with a nucleic acid, thus forming a conjugate or alternatively labeling the conjugate with a nucleic acid; amplifying the nucleic acid in the conjugate with DNA polymerase or RNA polymerase; and detecting or quantitating the amplified DNA or RNA. It is useful for the diagnosis of infectious diseases, autoimmune, hormonal abnormalities, metabolic abnormalities and cancers and the detection of histocompatibility antigens, hormones and bacterial toxins.

EP 0 544 212 A1

Rank Xerox (UK) Business Services
(3.10/3.6/3.3.1)

## BACKGROUND OF THE INVENTION

This invention relates to a highly sensitive method for detecting in a high sensitivity a trace substance from very small amounts of a test sample or quantitating it. This method can be utilized in the diagnosis of infectious diseases, autoimmune, hormonal abnormalities, metabolic abnormalities and cancers and the detection of histocompatibility antigens, hormones and bacterial toxins.

In the detection or quantitation of trace amounts of biological substances, a reaction between the biological substance and a substance specifically binding thereto has been favorably used as in immunoassay and receptor assay. They include labeling one of the ingredients participating in the reaction such as an object substance, a substance specifically binding thereto or a third substance specifically binding thereto with a radioactive substance and an enzyme, finally detecting the labeled substance thus reacted and quantitating it. In that case, the detection limit of the labelled substance is a major factor for deciding a sensitivity in the detection and quantitation of an object substance. The detection limit of labelled substances usually used in these methods are e.g., 1 fmol for a $^3H$ radioactive substance, 10 amol for an $^{125}I$ radioactive substance and 0.0002 amol (120 molecules) for $\beta$-galactosidase enzyme. This indicates that there is a theoretical limit to sensitivity in the detection and quantitation. The above-recited detection limits are for an exceptionally high sensitivity. In many cases, however, an affinity of a certain substance for a substance specifically binding thereto is not satisfactorily high.

An enzyme immunoassay, conventionally employing the amplification with an avidin-biotin system has the disadvantages of increased background and thus poor sensitivity due to a nonspecific binding of the biotinylated labelled substance or avidin. Therefore, a method with very high specificity has been employed in the operation for high sensitization to inhibit the interference of the background.

The polymerase chain reaction method (PCR method) is a method of annealing two primers across the specific DNA region acting as a template, repeating a reaction with DNA polymerase and amplifying specifically the template region (European Patent Application 0 200 362 A2). Honghua Li, et al have reported in Nature Vol. 335, pp. 414-417, 1988 that the PCR technique can provide $10^4$-$10^5$ fold amplification of a specific DNA region, which allows the detection of as little as a single molecule of DNA in a sample. Thus PCR is a very good method for the detection of nucleic acids such as DNA and RNA, which has widespread applications in gene diagnosis, virus diagnosis and detection of microorganisms using trace amounts of sample. However, there is a problem that the PCR method is unable to amplify other substances than nucleic acid, thus leading to inapplicability to the detection of those substances.

## SUMMARY OF THE INVENTION

It is therefore a general object of this invention to provide a highly sensitive method of detecting a trace substance from very small amounts of sample or quantitating the substance, which method is free of the disadvantages of the prior art processes with insufficient sensitivity to detect and quantitate trace amounts of biological substances, especially other substances than nucleic acid.

It is still more specific object of this invention to provide a simple method of detecting an object substance quickly and in high sensitivity, in which a nucleic acid is used as a label, a substance specifically binding to the object substance is labeled with the nucleic acid, the labeled substance is bound to the object substance and then the nucleic acid is enzymatically amplified.

The technique of the invention is applicable to all the techniques using a label substance and can be substituted for any of the prior art processes for the assay of trace biological substances by detecting or quantitating a labeled substance.

The present invention provides a method for the detection or quantitation of a trace substance in a sample which comprises:

(a) reacting a substance to be detected with a specifically binding substance which has a specific affinity thereto and is labeled with a nucleic acid, thus forming a conjugate; or alternatively

(a') reacting a substance to be detected with a specifically binding substance which has a specific affinity thereto, thus forming a conjugate, followed by labeling the conjugate with a nucleic acid;

(b) amplifying the nucleic acid in the conjugate with DNA polymerase or RNA polymerase; and

(c) detecting or quantitating the amplified DNA or RNA.

## BRIEF DESCRIPTION OF THE DRAWINGS

Figure 1 represents a photograph of ethidium bromide-stained bands produced according to the procedure mentioned in Example 1 below.

Figure 2 represents a photograph of ethidium bromide-stained bands produced according to the procedure mentioned in Example 2 below.

DETAILED DESCRIPTION OF THE INVENTION

The process of the invention utilizes a specific binding between ligand and acceptor or ligand and receptor such as antigen and antibody, substrate or coenzyme or its derivative and enzyme, saccharide chain and lectin, hormone and specific binding protein or the like. Thus the substances to be detected include hormones such as growth hormone, insulin, adrenocorticotropic hormone (ACTH), thyroid-stimulating hormone (TSH), luteinizing hormone (LH) and gut hormone, e.g., glicentin; growth factors such as epidermal growth factor (EGF), nerve growth factor (NGF), platelet-derived growth factor (PDGF), fibroblast growth factor (FGF), insulin-like growth factor (IGF) and hepatosite growth factor; bacterial toxins; bacterial metabolites and antibodies thereto; exosporium components; virus capsid components; enzymes such as alkaline phosphatase (ALP), glutamate-oxaloacetate transaminase (GOT), glutamate-pyruvate transaminase (GPT), lactate dehydrogenase (LDH), blood clotting factor, RNA polymerase and DNA polymerase; lipoproteins such as very low-density lipoprotein (VLDL), high-density lipoprotein (HDL) and low-density lipoprotein (LDL); receptors such as hormone receptors, e.g., insulin receptor, growth hormone receptor, EGF receptor and nerve receptor, e.g., acetylcholine receptor; cancer markers such as $\alpha$-fetoprotein (AFP), ferritin and carcinoembryonic antigen (CEA); cell surface antigens such as histocompatibility antigen; autoantibodies; c-reactive proteins; and other physiological active substances such as peptide enzyme inhibitors, e.g., pancreatic trypsin inhibitor, amylase inhibitor, $\alpha_2$ macroglobulin; and complements such as carrier protein, IGF-binding protein and transferrin. If the substances to be detected are for instance, hormones, the specifically binding substances are generally antibodies thereto and receptors.

In an embodiment of the present process, an object substance to be detected in a sample may be isolated by immobilizing on a solid carrier such as microplate, beads, disc piece and gel and subsequently to the immobilized object substance is bound a substance having a similar specific binding to said object substance and labeled with a nucleic acid. Alternatively, an object substance in a sample may be reacted with a substance having a specific binding to said object substance and labeled with a nucleic acid, thus forming a conjugate and subsequently immobilizing the conjugate. As a further option, a conjugate of an object substance with a specific binding substance may be immobilized and subsequently labeled with a nucleic acid. In all embodiments, the labeled nucleic acid as immobilized is amplified with DNA polymerase or RNA polymerase and the object substance in the sample is detected and quantitated from the amplified amounts of DNA or RNA. In that case, there is no problem whether or not the substance to be detected or quantitated contains a nucleic acid. If the object substance contains a nucleic acid, a base sequence not contained in the nucleic acid may be used for labeling. In any event, the nucleic acid used for labeling may be a size in the range from oligonucleotides to an order of kilo base units, but preferably the size of such an extent as not interfering with a specific binding between the labeled substance and the object substance.

The nucleic acid used for labeling may be either DNA or RNA. It may also be single or double stranded. The single-stranded RNA is easily digestible with a RNase activity in the test solution or the reaction solution for amplification. When the nucleic acid is used in a single strand, therefore, it requires generally DNA or a RNase-resistant RNA synthesized with a modified nucleotide. When the nucleic acid is used in a double strand, it may be either DNA or RNA. The nucleotide sequence of the nucleic acid used is not limited to specific one.

The labeling is performed by previously modifying a label nucleic acid in the process of its synthesis so that it can be bound to a biological ingredient such as protein, lipid or saccharide and then reacting the label with the substance to be labeled to bind them together. For example, a method of labeling is known which comprises intorducing an SH group at the 5' terminus of the nucleic acid and reacting the SH group with a maleimide group which has been introduced into a protein (Innis et al, PCR Protocols, pp. 99-112, Academic Press, 1990). Alternatively, said SH group and an SH group in the biological ingredient may be oxidized to form a disulfide bond. This bond is reversibly cleavable with a reducing agent and hence is convenient for the amplification reaction.

A specific and strong binding such as avidin-biotin binding can be utilized to bind a nucleic acid indirectly, i.e., through other material. For example, a substance specifically binding to an object substance to be detected is first biotinylated and then an excess of avidin is bound to the labeled biotin. One molecule of avidin is capable of binding to four molecules of biotin, but in the condition of avidin excess, the labeled biotin and avidin are bound at a molar ratio of 1:1, by which avidin bound to the labeled substance retains a binding capacity with further three molecules of biotin. The labeling with a nucleic acid may be performed via avidin by the reaction with a nucleic acid which has been biotinylated using a kit commercially available, e.g., from Milligen/Biosearch or Pharmacia. This process permits the labeling with a nucleic acid after the object substance and the specifically binding substance having a specific affinity thereto formed a conjugate. Thus even a high molecular nucleic acid can be used as a label with almost no influence on the formation of the conjugate.

Amplification of the labeled nucleic acid may be accomplished by a known PCR method (Saiki et al., 1985, Science 230, 1350-1354 & Saiki et al., 1988, Science, 239, 487-491) using Klenow DNA polymerase or Taq polymerase. In this process, nucleic acids and primers acting as a template are selected suitably to enable the sensitivity to amplify with no influence on the background. One cycle of the DNA polymerase reaction can double a DNA region. In principle, n cycles of the reaction may amplify the DNA to a size of $2^n$ times larger than the initial. Thus, repeated reaction can amplify the DNA region to a level of $10^5$ to $10^6$ times. Trace amounts of DNA present in a test sample can be quantitated by a suitable selection of the amount of a probe used for the reaction and cycles of the reaction. This enables the detection of as little as a single molecule of DNA.

Alternatively, an introduction into the labeled DNA of a sequence transcribed with RNA polymerase enables the amplification by transcription to RNA with the RNA polymerase.

It is not essential that the labeled nucleic acid is directly amplified as a template. For instance, a single-stranded oligonucleotide is used for labeling and a larger nucleic acid acting as a template can be hybridized for amplification. In that case, the label has satisfactorily a base number of such an extent that hybridization can be performed (from ten-odd to several tens). Alternatively, it is also possible to hybridize a DNA that is transcribed with RNA polymerase.

The amplification may be carried out with a conjugate of an object substance and a specifically binding label substance being immobilized on a solid carrier. Alternatively, it may be conducted after the template nucleic acid is liberated by digestion with a proteolytic enzyme such as pronase or protinase K. The liberation can be effected with a surface active agent such as sodium dodecylsulfate, sarcosine and Triton® (Rohm and Haas Co.) , or with chaotropic ions such as thiocyanate and calcium ions or with a heat, acid or alkali treatment, or with a reducing agent such as dithiothreitol and $\beta$-mercaptoethanol.

The detection of the amplified nucleic acid is performed by a method known per se, for example using a radioisotope, an enzyme or a fluorescent material. The methods for the detection include a direct process using a labeled primer in the amplification reaction or using a labeled substance for a precursor in the reaction solution and an indirect process of hybridizing the amplified nucleic acid or using a label having a specific affinity.

The invention is further illustrated by the following examples.

Example 1

Method using DNA-labeled antibody

Step 1: Introduction of a maleimide group into anti-glicentin antibody

Ten milligrams of polyclonal antibody (IgG fraction of rabbit antiserum) to glicentin (1-32) were subjected to affinity chromatography using glicentin as a ligand for the purification of the anti-glicentin antibody. The purified antibody was prepared in a concentration of 1 mg/0.3 ml with 20 mM sodium phosphate (pH 7.2). To the antibody was added 0.1 mg of a maleimide reagent (N-(maleimidomethyl)-cyclohexane-1-carboxylate) dissolved in 30 $\mu$l of N,N-dimethylformamide. The solution was allowed to react at 30°C for 1 hour. After the reaction insoluble matters were removed by centrifugation for 10 min., and unreacted maleimide reagent was removed by gel filtration through an NAP-25® column (Pharmacia) equilibrated with 0.1 M sodium phosphate (pH 6.0). Fractions each in a volume of 0.5 ml were measured for absorption at 280 nm. The peak fractions were collected.

Step 2: Preparation of label DNA

DNA was synthesized using a fully automatic DNA synthesizer Gene Assembler® (Pharmacia) in accordance with the $\beta$-cyanoethyl-phosphamidite method. SH groups were introduced at the final stage of the DNA synthesis into 5' termini of the synthesized DNA by the use of C6-Thiolmodifier (Amersham). The synthesized DNA has the following sequence

5' SH-CCGTGTATTC TATAGTGTCA CCTAAATCGT ATGTGTATGA TACATAAGGT

TATGTATTAA TTGTAGCCGC CGTTCT-OH 3'

Subsequently, cleaving of the DNA from the support, removal of the protective group and purification of the synthesized DNA were conducted according to the supplier's protocols. The purified DNA was subjected to gel filtration through an NAP-10® column (Pharmacia) equilibrated with 0.1 M sodium phosphate (pH 6.0) to prepare 1.5 ml of a 0.1 M sodium-phosphate solution (pH 6.0).

Step 3: Preparation of DNA-labeled antibody

The protein fraction obtained in Step 1 and the DNA solution obtained in Step 2 each in a volume of 1.5 ml were admixed. The mixture was allowed to react overnight at 4°C to form a DNA-antibody conjugate (DNA-labeled antibody). After centrifugation for 10 min. for removal of insoluble matters, the reaction mixture was subjected to ion-exchange column chromatography on a Mono Q® column (Pharmacia) to remove unreacted DNA and antibody. The conjugate was then collected in 2 ml fractions. Retention of the antibody activity in said conjugate was confirmed by a conventional enzyme-antibody method using a microplate on which glicentin was immobilized. Retention of DNA in said conjugate was confirmed by the following method. PCR amplification was performed by a conventional method using a primer having the same sequence as in the neighborhood of the 5' terminus of said DNA, 5'-GTATTCTATAGTGTCACCTA-3' and a primer complementary to the 3' terminus of said DNA, 5'-AGAACGGCGGCTACAATTAAT-3'. The amplified DNA was separated by gel electrophoresis from the primers and subjected to ethidium bromide staining to detect the amplified DNA.

Step 4: Immobilization of primary antibody

Polystyrene beads were mixed with mouse monoclonal antibody to the glicentin C terminus diluted with 50 mM sodium phosphate (pH 7.0) to a concentration of 10 $\mu$g/ml. The mixture was stirred at 4°C for 20 hours to immobilize the antibody. Blocking of said beads was made using Block Ace® (Dainippon Seiyaku) 1:4 diluted with the same phosphate buffer. After washing with PBS (10 mM sodium phosphate, pH 7.2, 0.15 M sodium chloride), the beads were stored in PBS until they were used.

Measurement

Following a similar procedure as in a conventional sandwich EIA method, a substance to be detected, glicentin and the labeled antibody were immobilized on the beads prepared in Step 4. In that case, the beads and 1 pg of recombinant human glicentin were allowed to react in a siliconized glass test tube. The beads were washed with PBS containing 0.05% Tween and subsequently reacted with the conjugate obtained in Step 3 as the secondary antibody. Then, the beads were washed to remove unreacted, labeled antibody and transferred to a polypropylene micro centrifuge tube. Thirty cycles of PCR amplification were carried out following the known method described in J. Sambrook et al, Molecular Cloning, 2nd ed. 1989. The amplified DNA was subjected to gel electrophoresis and confirmed by ethidium bromide staining.

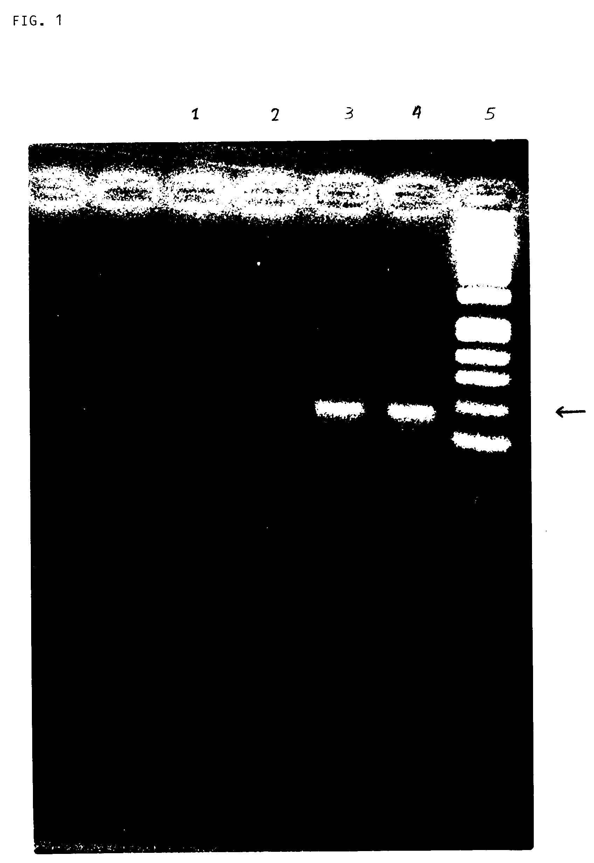

The results are shown in Fig. 1. Fig. 1 represents a photograph of ethidium bromide-stained bands produced when 1/10 in amount of the reaction products after PCR amplification of
    1. primer pair used in PCR
    2. detection system with no glicentin added
    3. detection system with 1 pg of glicentin added, and
    4. DNA used as a label,
    5. DNA size marker ($\phi$ x 174 DNA/Hinf I)
respectively, were subjected to electrophoresis on 4% agarose gel and staining with the reagent. The bands indicated by an arrow in the photograph show the DNA amplified by PCR.

This demonstrates that the method of the invention can detect trace amounts of glicentin which have not been detected by the EIA method.

Example 2

Labeling method using 5' biotinylated DNA

Step 1: Synthesis of biotinylated primer

A DNA primer was synthesized by the use of a fully automatic DNA synthesizer, Gene Assembler® (Pharmacia). The base sequence is 5'-ATCGTCCATTCCGACAGCAT-3', which is identical with the length of 20 bases of pBR322, a commercially available vector downstream of the restriction enzyme EcoR V site. The amino group for biotinylation was introduced at the final stage of DNA synthesis by means of N-MMT-hexanolamine linker (Milligen/Biosearch) into 5' terminus of the synthesized DNA. Biotinylation of the amino group at 5' terminus was carried out in the following manner. A solution of 5 mg of NHS-Biotin (Pierce Co., Ltd.) in 450 $\mu$l of DMF (dimethylformamide) was admixed with 120 $\mu$g/450 $\mu$l of the synthesized DNA, and the mixture was allowed to react overnight at room temperature. Unreacted biotin was removed by passing through NAP-10® and NAP-25® columns (Pharmacia) equilibrated with 20% ethanol.

Step 2: Preparation of biotinylated label DNA

A biotinylated DNA for labeling was prepared as described below. A PCR reaction was carried out using as a template a fraction of 192 base pair of commercially available vector pBR 322 digested with restriction enzymes EcoR V and BamH I and 100 pmol of the primer on the EcoR V side prepared in Step 1 and 5 pmol of a primer on the BamH I side with a base sequence of 5'-GATCCACAGGACGGGTGTGG-3' in 100 $\mu$l of Taq polymerase buffer in the presence of 2.5 units of Amplitaq® DNA polymerase (Takara Shuzo Co., Ltd.). The amplified DNA was confirmed by electrophoresis on 1% agarose gel. After the electrophoresis, DNA in the gel was transferred onto Immobilon® N (Millipore Co., Ltd.) for Southern blotting analysis and then incubated with avidin-HRP (horseraddish peroxidase) to bind it to the biotinylated DNA. Subsequently, OPD (orthophenylenediamine) which is a substrate for HRP was added, and the amplified DNA with the biotinylated DNA was detected by color development.

Measurement

As the antibodies to be immobilized on beads were used antibodies against an insulin-like growth factor binding protein. Following a similar procedure as in Example 1, said antibodies were allowed to react with an insulin-like growth factor binding protein to be detected and the insulin-like growth factor biotinylated previously with a biotinylation kit (Amersham Co., Ltd.) according to the supplier's protocols, thus forming a conjugate of antibody-substance to be detected-specifically binding substance. Subsequently, the beads were washed to remove unreacted biotinylated insulin-like growth factor. Avidin D 1:4000 diluted with PBS was then added to bind to the biotinylated insulin-like growth factor immobilized on the beads. Unbound avidin D was removed and the biotinylated DNA was added as label. The beads were thoroughly washed, the DNA was amplified in a similar manner as in Example 1 and subjected to detection procedures.

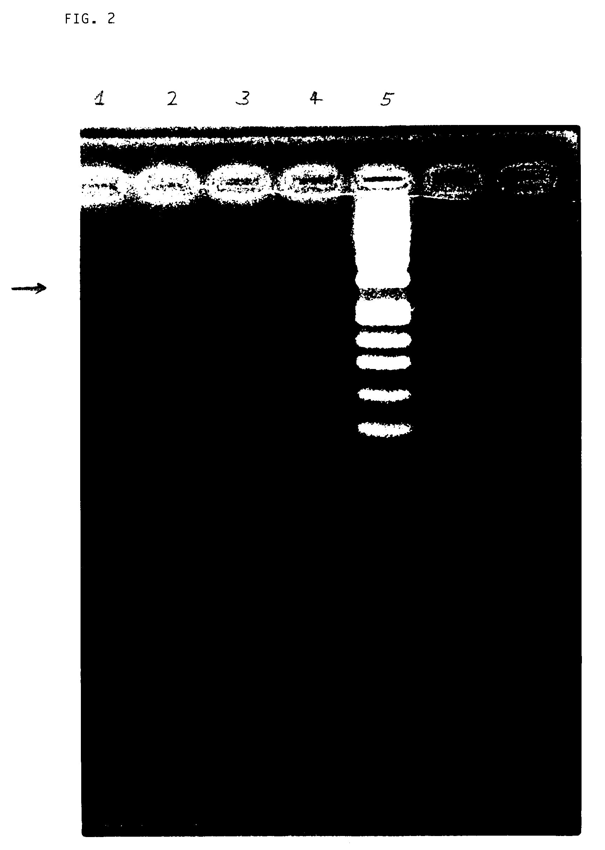

The results are shown in Fig. 2. Fig. 2 represents a photograph of ethidinium bromide-stained bands produced when 1/10 in amount of the reaction products after PCR amplification of

1 and 2. detection system with no insulin-like growth factor binding protein added
and

3 and 4. detection system with 50 pg of insulin-like growth factor binding protein added,

5. DNA size marker ($\phi$x174 DNA/Hinf I)

respectively, were subjected to electrophoresis on 4% agarose gel and staining with the reagent. The bands indicated by an arrow in the photograph show the DNA amplified by PCR.

This example demonstrates that a trace substance can be detected by utilization of a specific binding other than antigen-antibody binding such as one between hormone and protein specifically binding thereto. It also demonstrates that the detection is feasible when a conjugate formation is followed by nucleic acid labeling.

Example 3

Quantitation with DNA-labeled Fab'

Step 1: Preparation of anti-alkaline phosphatase Fab' fragment

IgG was purified from the antiserum against alkaline phosphatase disclosed in Japanese Patent 63-209597 A using Protein A column (BioRad Co., Ltd.) according to the supplier's protocols. An Fab' fragment was prepared from 10 mg of the IgG by a known procedure.

Step 2: Preparation of DNA-labeled Fab'

The Fab' as prepared and the DNA having an SH group at 5' terminus prepared according to the procedure of Example 1 were mixed in equimolar mixture. The mixture was allowed to react at room temperature for 20 hours to form a DNA-Fab' conjugate. The conjugate was purified in a similar manner as in Example 1. Formation of the conjugate was identified as follows: The conjugate was reacted with alkaline phosphatase which had been immobilized on polystyrene beads. Subsequently, it was subjected to PCR reaction after which the amplified DNA was detected for the identification of the conjugate.

Measurement

A similar procedure as in Step 4 of Example 1 was carried out using as an immobilized primary antibody, anti-alkaline phosphatase monoclonal antibody No. 21 and as a secondary antibody, the Fab' fragment of anti-alkaline phosphatase polyclonal antibody labeled with DNA. The beads were washed and then reacted in Taq polymerase buffer containing 1 mM of dithiothreitol at 37°C for 1 hour, thus liberating DNA. Then, PCR amplification was carried out according to the Makino's method described in Jikken Igaku (Experimental Medicine), vol. 9, pp. 103-106, 1991 using radioisotope, thereby enabling the quantitation of the amplified DNA.
The results are shown below.

| Alkaline phosphatase pg/assay | cpm |
|---|---|
| 0 | 17.0 |
| 0.01 | 39354 |
| 0.1 | 75384 |
| 1 | 126046 |
| 100 | 346842 |

INDUSTRIAL APPLICABILITY

The present invention provides a highly sensitive method for the detection and quantitation of trace substances which have hitherto been very difficult to detect due to its detection limits.
The method of the invention is useful for the diagnosis of infectious diseases with bacteria, viruses, parasites or the like by the detection of antigens such as exosporium component, toxin and intermediate or antibodies thereto; the diagnosis of autoimmune diseases by the detection of autoantibody or c-reactive protein; the diagnosis of hormonal or metabolic abnormalities by the detection of hormones such as growth hormone, insulin, epidermal growth factor, adrenocorticotropic hormone, thyroid-stimulating hormone or luteinizing hormone; the diagnosis of cancers by the detection of cancer marker such as α-fetoprotein, ferritin or carcinoembryonic antigen; a general biochemical blood examination of HDL, LDL, GOT, GPT or the like; and the detection of bacterial toxin.

**Claims**

1. A method for the detection or quantitation of a trace substance in a sample which comprises:
    (a) reacting a substance to be detected with a specifically binding substance which has a specific affinity thereto and is labeled with a nucleic acid, thus forming a conjugate or alternatively

7

(a') reacting a substance to be detected with a specifically binding substance which has a specific affinity thereto, thus forming a conjugate, followed by labeling the conjugate with a nucleic acid

(b) amplifying the nucleic acid in the conjugate with DNA polymerase or RNA polymerase; and

(c) detecting or quantitating the amplified DNA or RNA.

2. A method of Claim 1 comprising the steps of (a), (b) and (c).

3. A method of Claim 1 comprising the steps of (a'), (b) and (c).

4. A method of Claim 2 in which the substance to be detected is isolated by immobilizing on a solid carrier prior to step (a).

5. A method of Claim 2 in which the conjugate in step (a) is immobilized on a solid carrier.

6. A method of Claim 3 in which the conjugate in step (a') is immobilized on a solid carrier and then labeled with a nucleic acid.

7. A method of Claim 1 in which the substances to be detected are hormones, growth factors, bacterial toxins, bacterial metabolites and antibodies thereto, exosporium components, virus capsid components, enzymes, lipoproteins, receptors, cancer markers, cell surface antigens, autoantibodies, c-reactive proteins or other physiological active substances.

8. A method of Claim 7 in which the hormones are growth hormone, insulin, adrenocorticotropic hormone (ACTH), thyroid-stimulating hormone (TSH), luteinizing hormone (LH) or gut hormone, e.g., glicentin.

9. A method of Claim 7 in which the growth factors are epidermal growth factor (EGF), nerve growth factor (NGF), platelet-derived growth factor (PDGF), fibroblast growth factor (FGF), insulin-like growth factor (IGF) or hepatosite growth factor.

10. A method of Claim 7 in which the enzymes are alkaline phosphatase (ALP), glutamate-oxaloacetate transaminase (GOT), glutamate-pyruvate transaminase (GPT), lactate dehydrogenase (LDH), blood clotting factor, RNA polymerase or DNA polymerase.

FIG. 1

FIG. 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | Week 9135, Derwent Publications Ltd., London, GB; AN 91-256837 & JP-A-3 167 474 (HITACHI KK) 19 July 1991 * abstract * --- | 1-10 | C12Q1/68 G01N33/58 |
| X | WO-A-9 117 442 (CHIRON CORP.) * claims * --- | 1-10 | |
| A | EP-A-0 154 884 (MOLECULAR DIAGNOSTICS) --- | - | |
| A | GB-A-2 125 964 (CETUS CORP.) ----- | - | |
| | | | TECHNICAL FIELDS SEARCHED (Int. Cl.5 ) |
| | | | C12Q |

The present search report has been drawn up for all claims

| Place of search | Date of completion of the search | Examiner |
|---|---|---|
| THE HAGUE | 23 MARCH 1993 | MOLINA GALAN E. |

EPO FORM 1503 03.82 (P0401)